# EUROPEAN PATENT SPECIFICATION

(11) **EP 0 752 403 B1**
(45) Date of publication and mention of the grant of the patent: **03.01.2001**
(21) Application number: 96304930.9
(22) Date of filing: 04.07.1996
(51) Int. Cl.: C07C 17/358, C07C 21/073

(54) **Improved catalyst for the rearrangement of allylic geminal dihalogen compounds**
Verbessertes Katalysator für die Umlagerung von allylischen gem-dihalogenierten Verbindungen
Catalyseur amélioré pour le réarrangement de composés allyliques gem-dihalogénés

(30) Priority: 07.07.1995 US 499694
(43) Date of publication of application: 08.01.1997
(73) Proprietor: THE DOW CHEMICAL COMPANY, Midland, Michigan 48674 (US)
(72) Inventor: Ito, Larry N., Midland, Michigan 48640 (US)
(74) Representative: Burford, Anthony Frederick

(56) References cited:
- EP-A- 0 436 989
- US-A- 2 846 483
- US-A- 5 510 546
- PATENT ABSTRACTS OF JAPAN vol. 004, no. 106 (C-020), 30 July 1980 & JP 55 069523 A (SHOWA DENKO KK), 26 May 1980,

## Description

A significant by-product in the important commercial process of producing allyl chloride through the chlorination of propylene is 3,3-dichloropropene. Unfortunately, 3,3-dichloropropene and its homologs presently do not have a significant commercial use, so that the 3,3-dichloropropene produced in this manner has heretofore usually been incinerated. However, US-A-2,846,483 (published 5th August 1958 in pursuance of an application filed on 19th November 1953) did report that 3,3-dichloropropene had biocidal, especially nematocidal, activity and chemical utility, especially in hydrolysis to form acrolein, alcoholysis to form acetals, and reaction with alkaline sulfides or hydrosulfides to form thio-acrolein and related compounds. It was proposed in US-A-2,846,483 that 1,3-dichloropropene should be isomerized to 3,3-dichloropropene by heating, at least partially condensing the resultant vapors and fractionating the condensate.

As related in US-A-5,072,063 to Langensee (hereafter, "Langensee"), a number of efforts have been made at the same time to produce the 1,3-dichloropropenes also produced as byproducts in the allyl chloride process, because of the known utility of both the cis- and trans- isomers of 1,3-dichloropropene as, for example, nematocides (DE-A-1,210,618), soil fumigants, insecticides and monomers in the production of plastics, resins and chemical intermediates.

These past efforts have included reacting 1,2-dichloropropane with a gas containing oxygen in the presence of a catalyst containing CuCl₂, LiCl and ZnCl₂ at 470 to 490°C, dehydrochlorination of 1,2,3-trichloropropane in the presence of oxygen or halogen, contacting 1,2-dichloropropane with chlorine to effect both chlorination and dechlorination reactions, or mixing 1,2-dichloropropane with allyl chloride and/or 1-chloropropene and reacting with chlorine at high temperature.

Langensee elected, in view of the availability and lack of utility of 3,3-dichloropropene and further citing the complicated, energy-consuming and/or inconvenient nature of the aforementioned processes, to pursue the isomerization of a 3,3-dihalopropene generally and 3,3-dichloropropene more particularly to the respective 1,3-dihalopropenes or 1,3-dichloropropenes and homologs thereof. This isomerization was accomplished by contacting the 3,3-dichloropropene with an alumina, silica or zeolite catalyst, and especially an alumina, silica or zeolite having acidic sites, in preferably a fixed bed, continuous process. In particular, Langensee contemplated the isomerization would be conducted directly on a 3,3-dichloropropene-containing byproduct stream from a distillation of the product stream from an allyl chloride process, to produce cis- and trans-1,3-dichloropropenes to be combined with the cis- and trans-1,3-dichloropropenes otherwise produced in the allyl chloride process and recovered through distillation.

JP-A-80-69,523 was cited for also teaching an isomerization process, involving contacting 3,3-dichloropropene in the presence of hydrogen chloride with a catalytic amount of a zinc, iron, copper, tin, titanium or vanadium salt at between 0°C and 200°C. The difficulty found by Langensee with respect to the Japanese process, however, was that the catalyst was suspended in the reaction mixture and difficult to remove from the reaction mixture after completion of the rearrangement process.

The present invention provides a significant further improvement on the isomerization processes taught in Langensee and JP-A-80-69,523, and comprises a process for preparing the 1,3-dichloropropenes, such as cis- and trans-1,3-dichloropropene, and their homologs or the 1,3-dibromopropenes from 3,3-dichloropropene or 3,3-dibromopropene, respectively.

According to the present invention, there is provided a process for preparing a dihaloalkene compound of Formula I or II as hereinafter defined or a mixture of such compounds, which process comprises contacting a dihaloalkene compound of Formula I as hereinafter defined in the liquid state with an alumina, silica or zeolite catalyst, characterized in that the catalyst possesses a predominance of basic sites therein and has (a) a particle size in the range of from 0.15 mm to 12.5 mm and/or (b) an alkali or alkaline earth metal content of 0.1 to 10 weight percent.

The process of the present invention utilizes an alumina, silica or zeolite which is characterized in one embodiment by a particular range of particle sizes as well as by having a predominance of basic, as opposed to acidic, sites therein as associated with a high alkali or alkaline earth metals content, such aluminas, silicas and zeolites having been found to have unexpectedly superior lifetimes and productivities for the preferred isomerization of 3,3-dichloropropene to the useful 1,3-dichloropropenes as compared to the acidic aluminas, silicas and zeolites described and strongly favored by Langensee.

Further, and contrary to Langensee's teachings, there appears to be value in the context of the process of the present invention to conducting the process at higher temperatures. Langensee broadly teaches an isomerization process which is conducted at temperatures ranging from 0°C to 130°C, but prefers temperatures of from 20°C to 120°C, and especially prefers temperatures from 50°C to 110°C. The present, improved process is preferably conducted at elevated temperatures of from 120°C up to the degradation temperature of 1,3-dichloropropene, but more preferably is conducted at temperatures in excess of 130°C and most preferably is conducted at temperatures of 140°C and perhaps still higher temperatures.

Without being limiting of the present invention, it is theorized that the higher rates of deactivation observed below in an unmodified, acidic alumina, for example, may be attributed to an acid site-catalyzed polymerization of unsaturated chlorinated hydrocarbons in the allyl chloride byproduct stream, and that an exchange of sodium cations (or alkali or alkaline earth metals, more generally) at the acid sites makes these sites ineffective for such polymerization. Accordingly, the effect of exchanging alkali or alkaline earth metals at the acid sites of Langensee's favored, acidic alumina, silica or zeolite catalysts is expected to be most pronounced for the aluminas and zeolites as these are generally the more acidic of the three classes of materials, and less pronounced but still significant with the typically less-acidic silicas.

The materials to be isomerized by the present process are of the general Formula I as follows: 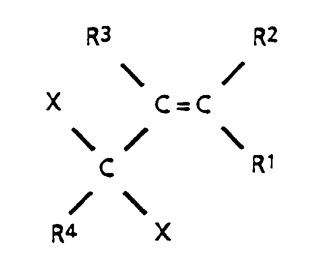 wherein X represents chloro and R¹, R², R³, and R⁴ independently represent hydrogen or a C₁-C₃ straight chain or branched chain alkyl group (methyl, ethyl, propyl and 1-methylethyl), or wherein X is bromo and each of R¹, R², R³, and R⁴ is hydrogen.

Compounds of this Formula I wherein X is chloro and R¹, R², R³ and R⁴ independently are hydrogen or a methyl group are preferred, as are compounds wherein each of R¹, R², R³ and R⁴ are hydrogen. Isomerization of 3,3-dichloropropene (3,3-DCPe) is especially of interest, such material being conventionally contained in an intermediate boiling point by-product fraction from the distillation of the product stream from a process of making allyl chloride by the chlorination of propylene, for example as disclosed in US-A-4,319,062 to Boozalis et al.

The intermediate boiling fraction that remains after removing a lower boiling fraction containing the desired allyl chloride product from such a process, and after removing higher boiling fractions containing most of the cis- and trans-1,3-dichloropropenes, includes a variety of chlorinated propanes and propenes of which generally between 10 and 20 percent by total weight is the targeted 3,3-dichloropropene. Other byproducts of the allyl chloride process include the just-mentioned cis- and trans-1,3-dichloropropenes, 2,3-dichloropropene, 2,2-dichloropropane, 1,2-dichloropropane and related species, with 1,2-dichloropropane being present generally as a major component at from 50 percent to 85 percent by weight, and typically being present at from 60 to 75 percent by weight.

The products of the isomerization process of the present invention in a general sense are the corresponding dichloroalkenes or dibromoalkenes of Formula II or Formula III: 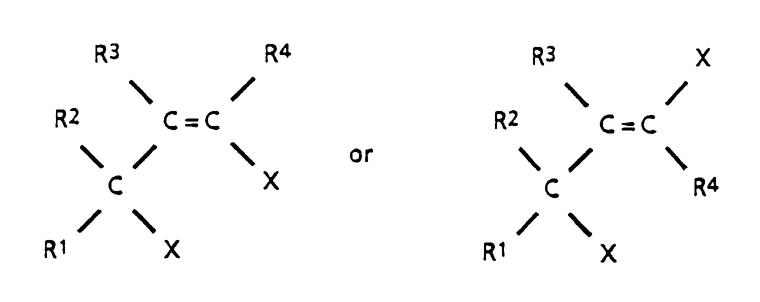 wherein X is chloro and R¹, R², R³ and R⁴ independently are hydrogen or a C₁-C₃ straight chain or branched chain alkyl group, or X is bromo and each of R¹, R², R³ and R⁴ is hydrogen. 1,3-Dichloropropene in either the cis- or trans-configuration is especially preferred as a product, corresponding to Formulas II and III, respectively, wherein X is chloro and each of R¹, R², R³ and R⁴ is hydrogen. Generally, some of both isomers is produced, and the present invention contemplates the production of either of these isomers or a mixture of these.

As has been indicated, a most preferred process embodiment will involve chlorinating propylene according to any conventional process for producing allyl chloride, then distilling the product stream to provide an intermediate boiling byproduct stream containing the 3,3-dichloropropene. Thereafter, the intermediate boiling byproduct stream containing 3,3-dichloropropene and what is in effect an inert chlorocarbon diluent is preferably exposed to an alumina, silica or zeolite catalyst which is characterized by particle sizes in the range of from 0.5 inches (12.5 mm) in diameter to 100 mesh (0.15 mm), as well as by having an alkali or alkaline earth metals content associated with a basic character in an aqueous slurry of the alumina, silica or zeolite, typically being from 0.1 weight percent to 10 weight percent on an elemental basis. Preferably, the alumina, silica or zeolite is characterized by a particle size of from 6 mesh (3.4 mm) to 40 mesh (0.4 mm), and most preferably is characterized by a particle size of from 10 mesh (2 mm), and especially 14 mesh (1.4 mm), down to 20 mesh (0.8 mm). A preferred alkali or alkaline earth metals content is from 0.2 weight percent to 5 weight percent, and most preferably the alkali or alkaline earth metal content of the catalyst will be from 0.3 weight percent to 2 weight percent.

Aluminas, silicas and zeolites are commercially available with various alkali metal contents, but with respect to the activated aluminas at least, those activated aluminas which are derived from Bayer process gibbsite, α-Al(oH)₃, or unrefined bauxite (containing as much as 90 percent of gibbsite on a dry basis) and which are characterized by comparatively higher amounts of sodium oxide (Na₂O) as an impurity (for example, 0.2 to 0.3 weight percent of Na₂O for gibbsite on a Al(OH)₃ basis) have been less favored for catalytic use (Kirk-Othmer, Encyclopedia of Chemical Technology, Vol. 2, "Aluminum Compounds (Activated)", 4th ed. (1995)), although apparently promoting aluminas with a "small amount" of alkali has been found beneficial in at least one other catalytic context, in certain Claus operations (Kirk-Othmer, citing a brochure, "SP-100 Promoted Activated Alumina for Claus Catalysis", Alcoa Chemicals Division, Aluminum Company of America, Pittsburgh, PA 1984). Aluminas with higher sodium oxide contents are consequently and advantageously inexpensive, and for this reason are especially preferred for use in the present invention. Aluminas which do not have the desired amounts of alkali or alkaline earth metals in their commercially-available forms may be impregnated with an aqueous solution of NaOH, for example, as an easy, inexpensive way of achieving the sodium/alkali metal content desired in the process of the present invention.

In a preferred embodiment of the invention, the catalyst is a basic alumina having a particle size in the range of from 0.4 mm to 3.4 mm and having an alkali or alkaline earth metal content of from 0.2 weight percent to 5 weight percent. Preferably, said catalyst is a basic, non-activated alumina having a particle size in the range of from 0.8 mm to 2 mm and having an alkali or alkaline earth metal content of from 0.3 weight percent to 2 weight percent.

Suitable commercially-available, basic aluminas which have been identified include Rhone Poulenc's SAS-350 activated alumina (Rhone Poulenc Inc., having a pH in aqueous slurry of about 9.3 and a sodium content of 0.6 weight percent on an elemental basis), Alcoa's F1 grade alumina (Aluminum Company of America, Pittsburgh, PA; not calcined/activated, having a pH in aqueous slurry of about 9.98 and a sodium content of 0.35 weight percent), Alcoa's F200 grade alumina (Aluminum Company of America, Pittsburgh, PA; not calcined/activated, having a pH in aqueous slurry of about 10.05 and a sodium content of 0.18 percent by weight), and Alcoa's DD2 grade alumina (having a pH in aqueous slurry of about 9.94). These are preferably ground and sieved as appropriate to be of the preferred particle sizes for use in the present invention.

Other than the departures from Langensee which have been discussed already (that is, in the selection of an alumina, silica or zeolite catalyst having a certain particle size and a certain alkali or alkaline earth metals content, and in terms of temperatures employed in the process), the process of the present invention can be satisfactorily carried out according to Langensee's teachings. Thus, the contemplated isomerization process can be carried out batchwise or continuously, with a fixed bed, continuous process being preferred but a fluidized bed process also being possible.

Any pressure can be used, but preferably the pressure and temperature are selected so that substantially all of the components of the byproduct stream fed to the isomerization process remain in the liquid state, exemplary pressures being from ambient pressure up to 1500 kPa. Reaction times can vary with the starting material to be isomerized, the process temperature, catalyst and reactor type employed. Exemplary batch times for a batchwise process range from 0.5 to 8 hours, generally falling in the range of from 1.5 to 4 hours. A fixed bed residence time in a continuous process of from 1 to 300 minutes is considered generally sufficient, with times of from 2 to 180 minutes, and especially from 2 to 120 minutes being preferred.

Where the 3,3-DCPe-containing, intermediate boiling byproduct stream is not processed directly through the isomerization process of the present invention, but is stored, transported or otherwise placed in an environment wherein the material may acquire water, then preferably the 3,3-DCPe-containing feed is first dried by any conventional means or method for accomplishing this end. In this regard, it has been found that water such as may be acquired by the feed material in storage or through other means has a significantly adverse impact on the rate of deactivation and productivity seen in further processing the stream according to the process described herein. Thus, the 3,3-DCPe-containing feed is preferably dried to a water content of less than 50 parts per million by weight, but more preferably is dried to a water content of less than 30 parts per million and most preferably 15 parts per million by weight or less of water.

The drying step can again be performed by any known means or according to any known method for doing so, a preferred, exemplary means or method involving passing the byproduct stream over molecular sieves or other water-absorbing materials conventionally used in drying applications at essentially ambient temperatures.

The present invention is more particularly illustrated by the Examples which follow:

### Example 1

An acidic activated alumina (pH of 6.54 in aqueous slurry, about 0.01 weight percent of sodium) sold by Norton Chemical Process Products with the designation SA-6275 was ground and sieved to a 14 by 20 mesh (1.4 mm by 0.85 mm), and impregnated with NaOH from aqueous solution to a sodium content of approximately 0.30 weight percent, on an elemental basis). The sodium-promoted alumina was then evaluated by passing 6.0 ml per hour of the 3,3-dichloropropene-containing, intermediate boiling fraction from a distillation of the products of a commercial allyl chloride process over 5.0 cubic centimeters of the alumina catalyst in a 316 stainless steel, 0.5 inch (12.7 mm) O.D. liquid phase tubular reactor maintained at 130 pounds per square inch, gauge (0.9 MPa, gauge) and 120°C. The maximum conversion observed of 3,3-dichloropropene to the cis- and trans- isomers of 1,3-dichloropropene was 99.1 percent, with an apparent deactivation rate of 0.00025 percent per hour and a productivity of 9,114 grams 3,3-DCPe (3,3-dichloropropene) converted per gram of the sodium-promoted alumina catalyst.

### Comparative Examples 1 and 2

For comparison, the same Norton SA-6275, acidic activated alumina was ground and sieved as in Example 1, and evaluated without being impregnated from an aqueous NaOH solution. All other conditions being the same, at a temperature of 90°C the maximum conversion observed was again at 99.1 percent, but the deactivation rate was 0.088 percent per hour and the productivity was 25.9 grams 3,3-DCPe converted per gram of catalyst. At a temperature of 120°C, the maximum conversion was 98.9 percent, the deactivation rate was 0.024 percent per hour, and the catalyst productivity was determined to be 94.9 grams of 3,3-DCPe converted per gram of catalyst.

### Examples 2 and 3

A basic alumina discussed and characterized above, grade SAS-350 activated alumina from Rhone Poulenc Inc., was ground and sieved to a 14 by 20 mesh (1.4 mm by 0.85 mm) and evaluated as in Example 1, at temperatures of 90°C and 120°C. At 90°C, the maximum conversion observed of 3,3-DCPe to 1,3-DCPe was 98.3 percent, with a deactivation rate of 0.00318 percent per hour and a productivity of 711 grams 3,3-DCPe converted per gram of catalyst. At 120°C, the maximum conversion was 98.8 percent, with a deactivation rate of 0.00127 percent per hour and with 1790 grams of 3,3-DCPe being isomerized per gram of catalyst.

### Comparative Example 3

For comparison to the results seen in Examples 2 and 3, and to illustrate the significance of particle size in the context of the exemplified process, a Rhone Poulenc grade SAS-350 activated alumina in the form of 3/16 inch (4.8 mm) spheres was evaluated at 120°C. The maximum conversion observed was 80.0 percent, with a deactivation rate of 0.038 percent per hour and a productivity of 23.5 grams 3,3-DCPe isomerized per gram of catalyst.

### Example 4

A basic, uncalcined Alcoa grade F1 alumina in an 8 by 14 mesh (2.4 mm x 0.85 mm) particle size was evaluated at 120°C in the manner of Examples 1-3. The maximum conversion was 99.1 percent, with a deactivation rate of 0.00123 percent per hour and a productivity of 1850 grams of 3,3-DCPe converted per gram of catalyst.

### Examples 5 and 6

A basic Alcoa DD2 grade alumina was ground and sieved to a 14 x 20 mesh (1.4 mm x 0.85 mm) size, and evaluated as in previous examples with a predried (with molecular sieves, to 15 ppm of water) feed at 90°C and 120°C. At 90°C, the maximum conversion was 98.5 percent, with a deactivation rate of 0.00324 percent per hour and a productivity of 698 grams of 3,3-DCPe converted per gram of alumina catalyst. At 120°C, the maximum conversion was 99.2 percent, the observed deactivation rate was 0.000926 percent per hour and the productivity was determined to be 2,460 grams of 3,3-DCPe converted per gram of catalyst.

### Examples 7 and 8

An acidic alumina from Calsicat (grade SB, having a pH in aqueous solution of 6.51 in an unimpregnated condition and being ground and sieved to a 14 x 20 mesh (1.4 mm x 0.85 mm) size) was impregnated with NaOH from an aqueous solution thereof to a sodium content of approximately 0.30 weight percent (on an elemental basis) and a pH of 10.35 in aqueous solution, and evaluated at 90 and 120°C. At 90°C, the maximum conversion was found to be 97.9 percent, with an observed deactivation rate of 0.0172 percent per hour and a productivity of 131 grams of 3,3-DCPe per gram of catalyst. At 120°C, the maximum conversion was 98.6 percent, the deactivation rate was 0.00491 percent per hour and the catalyst productivity was 461 grams of 3,3-DCPe feed converted per gram of catalyst.

### Comparative Examples 4 and 5

The same acidic alumina as employed in Examples 7 and 8 was run in an unimpregnated condition, with the result that at 90°C, the maximum conversion was 97.6 percent, with a deactivation rate of 0.321 percent per hour and a productivity of 6.99 grams of 3,3-DCPe converted per gram of catalyst. At 120°C, the maximum conversion was 98.4 percent, the deactivation rate was 0.0943 percent per hour and the productivity was found to be 23.9 grams of 3,3-DCPe converted per gram of catalyst.

### Examples 9 and 10

An acidic alumina from Norton Chemical Process Products (grade "9316079", with a pH in aqueous solution of 6.59 and being ground and sieved to a 14 x 20 mesh (1.4 mm x 0.85 mm) particle size) was impregnated with an aqueous solution of NaOH to a sodium content of approximately 0.30 weight percent, and to a pH in aqueous solution of 10.14. The impregnated catalyst was tested as in previous examples, at 90°C and 120°C. At 90°C, the maximum conversion was 97.5 percent, with a deactivation rate of 0.0836 percent conversion loss per hour and a productivity of 26.8 grams of 3,3-DCPe converted per gram of catalyst. At 120°C, the maximum conversion was found to be 98.4 percent, with a deactivation rate of 0.0246 percent per hour and a productivity of 91.8 grams of 3,3-DCPe converted per gram of catalyst.

### Comparative Examples 6 and 7

The same acidic alumina as employed in Examples 9 and 10 was run at 90 and 120°C, in an unimpregnated, acidic condition. At 90°C, the maximum conversion was 91.1 percent, the deactivation rate was considerably higher at 1.58 percent conversion loss per hour, and the productivity was 1.32 grams of 3,3-DCPe converted per gram of catalyst. At 120°C, the maximum conversion was 92.9 percent, the deactivation rate was 0.451 percent conversion loss per hour and the productivity was 4.72 grams of 3,3-DCPe converted per gram of catalyst.

The results from Examples 1-10 and Comparative Examples 1-7 are grouped and tabulated in Table 1 as follows:

**TABLE 1**

| Catalyst | T (°C) | Max.Conv.(%) | DR (% h) | Prod.(a) |
|---|---|---|---|---|
| Norton 6275 | 90 | 99.1 | 0.088 | 25.9 |
| | 120 | 98.9 | 0.024 | 94.9 |
| (Na⁺ added) | 120 | 99.1 | 0.00025 | 91114 |
| R-P SAS-350 | | | | |
| 3/16" | 120 | 80.0 | 0.038 | 23.5 |
| (4.8 mm) spheres | | | | |
| | | | | |
| SAS-350, | 90 | 98.3 | 0.00318 | 711 |
| 14 x 20 mesh | 120 | 98.8 | 0.00127 | 1790 |
| (1.4x0.85 mm) | | | | |
| Alcoa F-1 | 120 | 99.1 | 0.00123 | 1850 |
| Alcoa DD2 | 90 | 98.5 | 0.00324 | 698 |
| | 120 | 99.2 | 0.000926 | 2460 |
| Calsicat SB (Na⁺ added) | 90 | 97.6 | 0.321 | 6.99 |
| | 120 | 98.4 | 0.0943 | 23.9 |
| | 90 | 97.9 | 0.0172 | 131 |
| | 120 | 98.6 | 0.00491 | 461 |
| Norton 9316079 (Na⁺ added) | 90 | 91.1 | 1.58 | 1.32 |
| | 120 | 92.9 | 0.451 | 4.72 |
| | 90 | 97.5 | 0.0836 | 26.8 |
| | 120 | 98.4 | 0.0246 | 91.8 |

| | | | | |
|---|---|---|---|---|
| (a) Productivity in grams of 3,3-DCPe converted per gram of catalyst; | | | | |

As can be seen, particularly from a comparison of the results for the acidic aluminas tested in a conventional, unimpregnated condition and in a sodium-impregnated, basic condition, the basic versions of these aluminas exhibited surprisingly lower rates of deactivation and much higher productivities than the acidic versions favored by Langensee. For example, for the Norton 6275 grade alumina, at 120°C the deactivation rate of the acidic alumina was 96 times greater than for the sodium-impregnated, basic alumina formed therefrom. For the Calsicat SB grade alumina, the acidic, unimpregnated alumina deactivated 18.7 times as fast as the basic version at 90°C and 19.2 times as fast at 120°C. With respect to the Norton 9316079 grade alumina, the acidic alumina deactivated about 18.9 times as fast as the basic alumina formed therefrom at 90°C, and about 18.3 times as fast at 120°C.

## Claims

1. A process for preparing a dihaloalkene compound of the formula 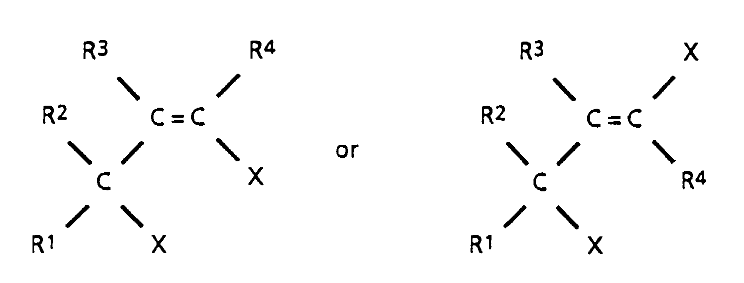 wherein
X represents chloro or bromo
R¹, R², R³, and R⁴ independently represent hydrogen or a C₁-C₃ alkyl group, with the proviso that when X represents bromo each of R¹, R², R³, and R⁴ represents hydrogen or a mixture of such compounds, which process comprises contacting a dihaloalkene compound of the formula 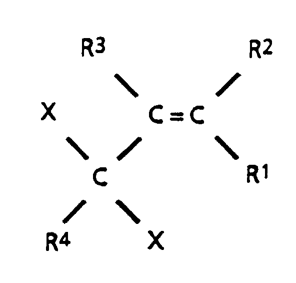 wherein X, R¹, R², R³, and R⁴ are as defined above in the liquid state with an alumina, silica or zeolite catalyst, characterized in that the catalyst possesses a predominance of basic sites therein and has (a) a particle size in the range of from 0.15 mm to 12.5 mm and/or (b) an alkali or alkaline earth metal content of 0.1 to 10 weight percent.

2. A process as claimed in Claim 1, wherein the catalyst has a particle size in the range 0.15 mm to 12.5 mm.

3. A process as claimed in Claim 1 for preparing cis- or trans-1,3-dichloropropene or a mixture of these, which comprises contacting 3,3-dichloropropene in the liquid state with an alumina, silica or zeolite catalyst which is characterized by an alkali or alkaline earth metals content of from 0.1 weight percent to 10 weight percent.

4. A process as claimed in Claim 1 or Claim 2, wherein the dihaloalkene compound contacted with the catalyst is 3,3-dichloropropene.

5. A process as claimed in Claim 3 or Claim 4, wherein the 3,3-dichloropropene starting material is a component of an intermediate boiling byproduct stream derived from the distillation of the products of a process for making allyl chloride by the chlorination of propylene.

6. A process as claimed in any one of the preceding claims, wherein the catalyst has an alkali metal or alkaline earth metal content of from 0.1 weight percent to 10 weight percent on an elemental basis.

7. A process as claimed in Claim 6, wherein the alkali or alkaline earth metal content is from 0.2 weight percent to 5 weight percent.

8. A process as claimed in Claim 7, wherein the alkali or alkaline earth metal content is from 0.3 weight percent to 2 weight percent.

9. A process as claimed in any one of the preceding claims, wherein the catalyst has a particle size in the range of from 0.4 mm to 3.4 mm.

10. A process as claimed in Claim 9, wherein the particle size is in the range of from 0.8 mm to 2.0 mm.

11. A process as claimed in any one of the preceding claims, wherein the process is conducted at a temperature in excess of 120°C.

12. A process as claimed in Claim 11, wherein the temperature is in excess of 130°C.

13. A process as claimed in Claim 12, wherein the temperature is at least 140°C.

14. A process as claimed in any one of the preceding claims, wherein the catalyst is a basic, non-activated alumina.

15. A process as claimed in any one of the preceding claims, wherein the catalyst is a basic alumina having a particle size in the range of from 0.4 mm to 3.4 mm and having an alkali or alkaline earth metal content of from 0.2 weight percent to 5 weight percent.

16. A process as claimed in Claim 15, wherein the catalyst is a basic, non-activated alumina having a particle size in the range of from 0.8 mm to 2 mm and having an alkali or alkaline earth metal content of from 0.3 weight percent to 2 weight percent.

17. The use of a catalyst selected from basic aluminas, silicas and zeolites as defined in any one of Claim 1 to 3, 6 to 10, or 14 to 16 to lower the rate of catalyst deactivation in an alumina, silica or zeolite-catalyzed isomerization of a 3,3-dihalopropene compound as defined in Claim 1 to a 1,3-dihalopropene compound as defined in Claim 1.

18. The use of a catalyst selected from basic aluminas, silicas and zeolites as defined in any one of Claim 1 to 3, 6 to 10, or 14 to 16 to improve the productivity in an alumina, silica or zeolite-catalyzed isomerization of a 3,3-dihalopropene compound as defined in Claim 1 to a 1,3-dihalopropene compound as defined in Claim 1.

## Patentansprüche

1. Verfahren zur Herstellung einer Dihalogenalkenverbindung der Formel 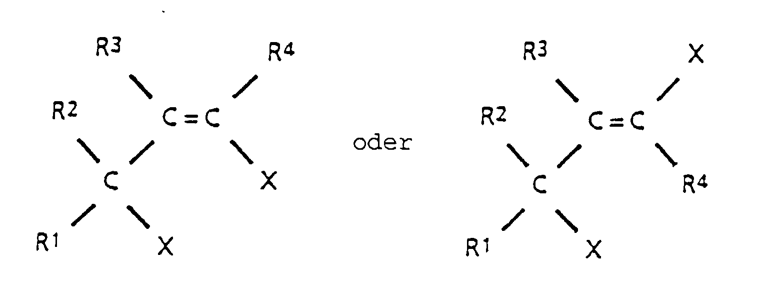 worin
X Chlor oder Brom bedeutet,
R¹, R², R³ und R⁴ unabhängig Wasserstoff oder eine C₁-C₃-Alkylgruppe bedeuten, mit der Maßgabe, daß wenn X Brom bedeutet, jedes von R¹, R², R³ und R⁴ Wasserstoff bedeutet oder eines Gemisches derartiger Verbindungen, wobei das Verfahren das Inkontaktbringen einer Dihalogenalkenverbindung der Formel 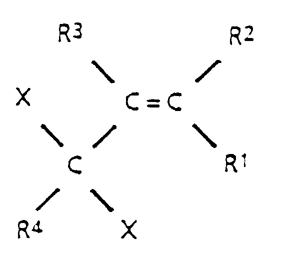 worin X, R¹, R², R³ und R⁴ wie oben definiert sind, im flüssigen Zustand mit einem Aluminiumoxid-, Siliciumoxid- oder Zeolithkatalysator umfaßt, dadurch gekennzeichnet, daß der Katalysator überwiegend basische Stellen darin aufweist und (a) eine Teilchengrösse im Bereich von 0,15 mm bis 12,5 mm und/oder (b) einen Alkali- oder Erdalkalimetallgehalt von 0,1 bis 10 Gew.-Prozent aufweist.

2. Verfahren nach Anspruch 1, worin der Katalysator eine Teilchengrösse im Bereich von 0,15 mm bis 12,5 mm aufweist.

3. Verfahren nach Anspruch 1 zum Herstellen von cis- oder trans-1,3-Dichlorpropen oder einem Gemisch von diesen, umfassend das Inkontaktbringen von 3,3-Dichlorpropen im flüssigen Zustand mit einem Aluminiumoxid-, Siliciumoxid- oder Zeolithkatalysator, der gekennzeichnet ist durch einen Alkali- oder Erdalkalimetallgehalt von 0,1 Gew.-Prozent bis 10 Gew.-Prozent.

4. Verfahren nach Anspruch 1 oder 2, worin die Dihalogenalkenverbindung, die mit dem Katalysator in Kontakt gebracht wird, 3,3-Dichlorpropen ist.

5. Verfahren nach Anspruch 3 oder 4, worin das 3,3-Dichlorpropenausgangsmaterial eine Komponente eines im Zwischenbereich siedenden Nebenproduktstroms ist, der aus der Destillation der Produkte eines Verfahrens zur Herstellung von Allylchlorid durch die Chlorierung von Propylen stammt.

6. Verfahren nach einem der vorhergehenden Ansprüche, worin der Katalysator einen Alkali- oder Erdalkalimetallgehalt von 0,1 Gew.-Prozent bis 10 Gew.-Prozent auf Elementbasis aufweist.

7. Verfahren nach Anspruch 6, worin der Alkali- oder Erdalkalimetallgehalt von 0,2 Gew.-Prozent bis 5 Gew.-Prozent ist.

8. Verfahren nach Anspruch 7, worin der Alkali- oder Erdalkalimetallgehalt von 0,3 Gew.-Prozent bis 2 Gew.-Prozent ist.

9. Verfahren nach einem der vorhergehenden Ansprüche, worin der Katalysator eine Teilchengrösse im Bereich von 0,4 mm bis 3,4 mm aufweist.

10. Verfahren nach Anspruch 9, worin die Teilchengrösse im Bereich von 0,8 mm bis 2,0 mm ist.

11. Verfahren nach einem der vorhergehenden Ansprüche, worin das Verfahren bei einer Temperatur über 120 °C durchgeführt wird.

12. Verfahren nach Anspruch 11, worin die Temperatur über 130 °C ist.

13. Verfahren nach Anspruch 12, worin die Temperatur mindestens 140 °C ist.

14. Verfahren nach einem der vorhergehenden Ansprüche, worin der Katalysator ein basisches nichtaktiviertes Aluminiumoxid ist.

15. Verfahren nach einem der vorhergehenden Ansprüche, worin der Katalysator ein basisches Aluminiumoxid mit einer Teilchengrösse im Bereich von 0,4 mm bis 3,4 mm ist und einen Alkali- oder Erdalkalimetallgehalt von 0,2 Gewichtsprozent bis 5 Gewichtsprozent aufweist.

16. Verfahren nach Anspruch 15, worin der Katalysator ein basisches nichtaktiviertes Aluminiumoxid mit einer Teilchengrösse von 0,8 mm bis 2 mm ist und einen Alkali- oder Erdalkalimetallgehalt von 0,3 Gewichtsprozent bis 2 Gewichtsprozent aufweist.

17. Verwendung eines Katalysators, ausgewählt aus basischen Aluminiumoxiden, Siliciumoxiden und Zeolithen wie in einem der Ansprüche 1 bis 3, 6 bis 10 oder 14 bis 16 definiert, um die Rate der Katalysatordeaktivierung in einer durch Aluminiumoxid, Siliciumoxid oder Zeolith katalysierten Isomerisierung einer 3,3-Dihalogenpropenverbindung, wie in Anspruch 1 definiert, in eine 1,3-Dihalogenpropenverbindung, wie in Anspruch 1 definiert, zu verringern.

18. Verwendung eines Katalysators, ausgewählt aus basischen Aluminiumoxiden, Siliciumoxiden und Zeolithen wie in einem der Ansprüche 1 bis 3, 6 bis 10 oder 14 bis 16 definiert, um die Produktivität bei einer durch Aluminiumoxid, Siliciumoxid oder Zeolith katalysierten Isomerisierung einer 3,3-Dihalogenpropenverbindung, wie in Anspruch 1 definiert, in eine 1,3-Dihalogenpropenverbindung, wie in Anspruch 1 definiert, zu verbessern.

## Revendications

1. Procédé pour préparer un dihalogénoalcène de formule : 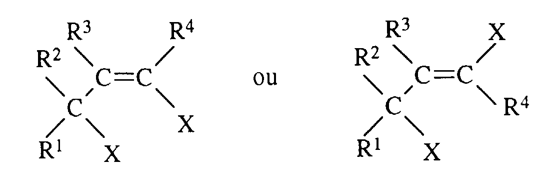 dans laquelle :
X représente un atome de chlore ou de brome,
R¹, R², R³ et R⁴ représentent indépendamment un atome d'hydrogène ou un groupe alkyle en C₁-C₃, à condition que quand X représente un atome de brome, R¹, R², R³ et R⁴ représentent chacun un atome d'hydrogène,
ou un mélange de tels composés, lequel procédé comprend la mise en contact d'un dihalogénoalcène de formule : 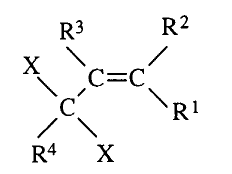 dans laquelle X, R¹, R², R³ et R⁴ sont tels que définis ci-dessus, à l'état liquide avec un catalyseur de type alumine, silice ou zéolithe, caractérisé en ce que le catalyseur possède à l'intérieur une prédominance de sites basiques et présente (a) une taille de particules se situant dans l'intervalle allant de 0,15 mm à 12,5 mm et/ou (b) une teneur en métal alcalin ou alcalino-terreux de 0,1 à 10 % en poids.

2. Procédé selon la revendication 1, dans lequel le catalyseur présente une taille de particules se situant dans l'intervalle allant de 0,15 mm à 12,5 mm.

3. Procédé selon la revendication 1, pour préparer le cis-1,3-dichloropropène ou le trans-1,3-dichloropropène ou un mélange de ces derniers, qui comprend la mise en contact du 3,3-dichloropropène à l'état liquide avec un catalyseur de type alumine, silice ou zéolithe qui est caractérisé par une teneur en métal alcalin ou alcalino-terreux allant de 0,1 % en poids à 10 % en poids.

4. Procédé selon la revendication 1 ou 2, dans lequel le dihalogénoalcène mis en contact avec le catalyseur est le 3,3-dichloropropène.

5. Procédé selon la revendication 3 ou 4, dans lequel le matériau de départ qui est le 3,3-dichloropropène est un composant d'un courant intermédiaire de sous-produits en ébullition provenant de la distillation des produits d'un procédé pour fabriquer du chlorure d'allyle par chloration du propylène.

6. Procédé selon l'une quelconque des précédentes revendications, dans lequel le catalyseur présente une teneur en métal alcalin ou en métal alcalino-terreux allant de 0,1 % en poids à 10 % en poids.

7. Procédé selon la revendication 6, dans lequel la teneur en métal alcalin ou en métal alcalino-terreux va de 0,2 % en poids à 5 % en poids.

8. Procédé selon la revendication 7, dans lequel la teneur en métal alcalin ou en métal alcalino-terreux va de 0,3 % en poids à 2 % en poids.

9. Procédé selon l'une quelconque des précédentes revendications, dans lequel le catalyseur présente une taille de particules se situant dans l'intervalle allant de 0,4 mm à 3,4 mm.

10. Procédé selon la revendication 9, dans lequel la taille de particules se situe dans l'intervalle allant de 0,8 mm à 2,0 mm.

11. Procédé selon l'une quelconque des précédentes revendications, dans lequel le procédé est mené à une température dépassant 120 °C.

12. Procédé selon la revendication 11, dans lequel la température dépasse 130 °C.

13. Procédé selon la revendication 12, dans lequel la température vaut au moins 140 °C.

14. Procédé selon l'une quelconque des précédentes revendications, dans lequel le catalyseur est une alumine basique non-activée.

15. Procédé selon l'une quelconque des précédentes revendications, dans lequel le catalyseur est une alumine basique présentant une taille de particules qui se situe dans l'intervalle allant de 0,4 mm à 3,4 mm et présentant une teneur en métal alcalin ou en métal alcalino-terreux allant de 0,2 % en poids à 5 % en poids.

16. Procédé selon la revendication 15, dans lequel le catalyseur est une alumine basique non-activée présentant une taille de particules qui se situe dans l'intervalle allant de 0,8 mm à 2 mm, et présentant une teneur en métal alcalin ou en métal alcalino-terreux allant de 0,3 % en poids à 2 % en poids.

17. Utilisation d'un catalyseur choisi parmi les silices, zéolithes et alumines basiques, tel que défini dans l'une quelconque des revendications 1 à 3, 6 à 10 ou 14 à 16, pour diminuer la vitesse de désactivation du catalyseur dans une isomérisation catalysée par de l'alumine, de la silice ou une zéolithe, d'un 3,3-dihalogénopropène tel que défini dans la revendication 1 en un 1,3-dihalogénopropène tel que défini dans la revendication 1.

18. Utilisation d'un catalyseur choisi parmi les silices, zéolithes et alumines basiques, tel que défini dans l'une quelconque des revendications 1 à 3, 6 à 10, ou 14 à 16, pour améliorer la productivité dans une isomérisation catalysée par de l'alumine, de la silice ou une zéolithe, d'un 3,3-dihalogénoalcène tel que défini dans la revendication 1 en un 1,3-dihalogénopropène tel que défini dans la revendication 1.
